# EUROPEAN PATENT SPECIFICATION

(11) **EP 2 352 463 B1**
(45) Date of publication and mention of the grant of the patent: **05.09.2012**
(21) Application number: 09764812.5
(22) Date of filing: 03.12.2009
(51) Int. Cl.: A61F 2/84

(54) **RETRACTABLE CATHETER**
ZURÜCKZIEHBARER KATHETER
CATHÉTER RÉTRACTABLE

(30) Priority: 03.12.2008 GB 0822109; 03.12.2008 US 119661 P
(43) Date of publication of application: 10.08.2011
(73) Proprietor: Angiomed GmbH & Co. Medizintechnik KG, 76227 Karlsruhe (DE)
(72) Inventor: WÜBBELING, Martin, 68161 Mannheim (DE); TÖLLNER, Thomas, 12047 Berlin (DE)
(74) Representative: HOFFMANN EITLE
(86) International application number: PCT/EP2009/066313
(87) International publication number: WO 2010/063793

(56) References cited:
- WO-A2-00/41525
- WO-A2-03/002019
- WO-A2-2006/113869
- WO-A2-2008/031103
- US-A- 5 534 007
- US-A1- 2006 247 661

## Description

### Field of the Invention

This invention relates to a retractable catheter preferably for use as an outer sheath in an implant catheter delivery system, and to a method of manufacturing a retractable catheter. Such a catheter is preferably sized to be inserted in a bodily lumen. Such a retractable catheter includes a pull element connected at its distal end to an elongate tubular sheath.

### Background Art

Conventional implant catheter delivery systems comprise an inner shaft that extends from a proximal end to a distal end of the system and carries on its distal end a self expanding implant. A longitudinal tubular sheath lies radially outside the implant to retain the implant in the catheter delivery system until the self expanding stent is to be released.

In WO 2006/133959, which is incorporated by reference in its entirety into this application, there is generally shown a catheter device having such a construction. During a medical procedure, the catheter device is pushed distally into a bodily lumen until the implant is disposed at a predetermined delivery position. To allow the implant to expand at the delivery position, the outer sheath has to be retracted. For this reason the sheath is connected to a pull wire which is pulled by means of a device to apply a pull force to the wire and, thus, to the outer sheath. To connect the wire to the outer sheath, commonly there is used an outer ring and an inner ring, the outer ring radially outside the outer sheath and being crimped or swaged onto the inner ring which is radially inside the outer sheath. The wire can then be connected to the inner ring, while the outer ring is clamped onto the inner ring, fastening both rings and the wire onto the sheath. When the wire is pulled, the sheath is retracted from where it surrounds the implant and the inner shaft, releasing the implant to expand.

Another possibility shown in WO 2006/133959 for fastening the wire relative to the outer sheath is to provide two outer rings outside the sheath, one on each side of an inner ring inside the sheath, to which the pull wire is fastened. The two outer rings are clamped onto the outer sheath, one proximal and one distal to the inner ring, to prevent relative movement between the outer sheath and the inner ring. In this way the outer sheath is connected to the pull wire by clamping it on either side of the inner ring, to move with the inner ring when the pull wire is retracted.

The implants which are to be delivered can be of a variety of lengths, and some may be quite long, for example up to 120 mm in length and 10 mm in diameter. With a longer implant, due to the higher friction forces between the greater contact surface area of the longer implant and the outer sheath, the retracting force needed to retract the outer sheath will necessarily increase with the length. This is particularly noticeable for covered stent grafts, where the friction with the outer sheath is large, as compared with an uncovered stent. Since a greater force has to be applied, the possibility arises that the inner shaft may deflect or move under compression, as tension is applied to the pull wire. The thickness of the inner shaft then must increase to withstand the compressive forces. Larger forces will also tend to break the connection between the pull wire and sheath.

Another disadvantage of such an arrangement is that the diameter of the outer sheath has to be at least large enough, for the inner shaft, the stent and the inner ring to be accommodated in the sheath. Furthermore, the outer rings increase the thickness of the device as well, at least if one is clamped directly on the inner ring, and may distort the outside surface of the outer sheath. Since an application of such an implant catheter delivery system is for delivery of stents by advancing the catheter system into a bodily lumen, a thicker diameter reduces the range of possible treatment opportunities, especially in the narrow confines of a patient's vasculature, or may give rise to a risk of injury as the catheter system is advanced.

US 5 534 007 A discloses a stent deployment catheter with collapsible sheath, but fails to disclose the characterizing features of independent Claims 1 and 10.

### Summary of the Invention

According to a first aspect of the present invention as disclosed in claim 1, there is provided a retractable catheter preferably for use in an implant delivery catheter system, the catheter comprising: an elongate tubular sheath which is at least in a distal portion made of a relatively flexible material; a reinforcing structure which is made of a relatively inextensible material and which is incorporated, along at least a portion of the length of the sheath, in the relatively flexible material of the sheath; and a pull element for transmitting a pull force longitudinally to the sheath and the reinforcing structure, characterized in that: the pull element is connected directly to a portion of the reinforcing structure exposed from the relatively flexible material, wherein the exposed portion of the reinforcing structure is exposed in an opening in the flexible material, to transmit the pull force directly to the reinforcing structure.

An advantage achievable with embodiments of the present invention is to improve the connection between a pull element and an outer sheath in a catheter system, whilst preferably avoiding an increase in the diameter of the outer sheath.

Another advantage obtainable with embodiments of the present invention is to provide an outer sheath having evenly formed outer and inner surfaces.

Due to the force being transmitted directly into the element of the catheter sheath which is strongest in tension, a greater force can be applied to the catheter without risk of damage to the catheter sheath or of breaking the connection between the pull element and the sheath. Application of tensile forces up to 110N has been achieved with this connection method, as compared with 31N for known swaging ring techniques. Furthermore, the need for swaged inner and outer rings can be obviated, which can reduce the overall retractable catheter diameter.

A further advantage which can be achieved is to provide a larger gap between the catheter sheath and an implant retained thereby, to stop the sheath pressing on the implant as it passes around a tight curvature, without any significant increase in the overall catheter diameter, due to the lower profile sheath that can be used with this construction. This can reduce the friction between the sheath and the implant during retraction, thus lowering the retraction force which must be applied. This enables deployment of longer or covered implants into relatively narrow body passages.

The opening can be provided at the best place for making the exposure and connection, increasing the flexibility and adaptability of the connection process.

In preferred embodiments, the reinforcing structure comprises a braiding. Braiding can provide well defined directional reinforcement of the sheath material, and can be securely encapsulated in the sheath material.

Preferably, the pull element is thermally connected to the reinforcing structure, the thermal connection method preferably including laser welding, resistance welding, gap welding, and/or point welding. Such a connection is easy to accomplish and further avoids unnecessary extra elements. Thus, the connection can be made thin, allowing more space within the sheath to slide past the implant, and friction during sheath retraction can be further reduced.

Preferably, the pull element is provided with at least one connection link by which it is connected to the reinforcing structure. The connection link may have an at least partially tubular element to support the connection to the reinforcing structure.

In preferred embodiments, at least a portion of the pull element is partially tubular, preferably at its distal end. Alternatively, the pull element is, at least at its distal end, tube shaped.

In further preferred embodiments, the reinforcing structure and/or the pull element and/or the connection link comprise steel, stainless steel, plastic, a carbon fibre compound and/or a glass fibre compound.

There is further provided an implant delivery system comprising a retractable catheter according to the first aspect of the invention, the delivery system further comprising an inner catheter shaft and a implant.

According to a second aspect of the present invention as disclosed in claim 10, there is provided a method for assembling a retractable catheter for use in a implant delivery system, comprising the step of:
- providing a catheter sheath made of relatively flexible material in which a relatively inextensible reinforcing structure is incorporated, and characterized by the steps of:
   - partially removing the relatively flexible material of the catheter sheath to expose a portion of the reinforcing structure to which a pull element is to be directly connected; and - connecting a pull element directly to an exposed portion of the reinforcing structure.

The removal of the relatively flexible material may be by thermal means, preferably using a laser.

In further preferred embodiments, the method further comprises the steps of: - providing the pull element with a connection link; and - connecting the connection link of the pull element directly to the reinforcing structure.

Preferably, the connection of the pull element to the reinforcing structure is made by laser welding and/or point welding.

In more preferred embodiments, the method further comprises the step of: - covering the exposed portion of the reinforcing structure after the connection of the pull element thereto.

Such methods may be used to assemble a retractable catheter or implant delivery device according to the first aspect of the invention.

### Brief Description of the Drawings

To enable a better understanding of the present invention, and to show how the same may be carried into effect, reference will now be made, by way of example only, to the accompanying drawings, in which:-
Fig. 1 shows a distal end of an embodiment of a retractable catheter according to the present invention;
Figs. 2a and 2b show a distal end of another example of a catheter and possible arrangement to connect the reinforcing structure with the pull element;
Fig. 3 is a perspective view of a distal end of an embodiment of a catheter according to the present invention;
Figs. 4a to 4h are different examples of an end portion which can be used in embodiments of the present invention; and
Figs. 5a and 5b show an embodiment in which the end portion of a pull element is embedded into the wall of an outer sheath which is provided with a braided reinforcing layer.

### Detailed Description

In the following, the expressions "distal", meaning away from the operator's side, and "proximal", meaning towards the operator's side, are used.

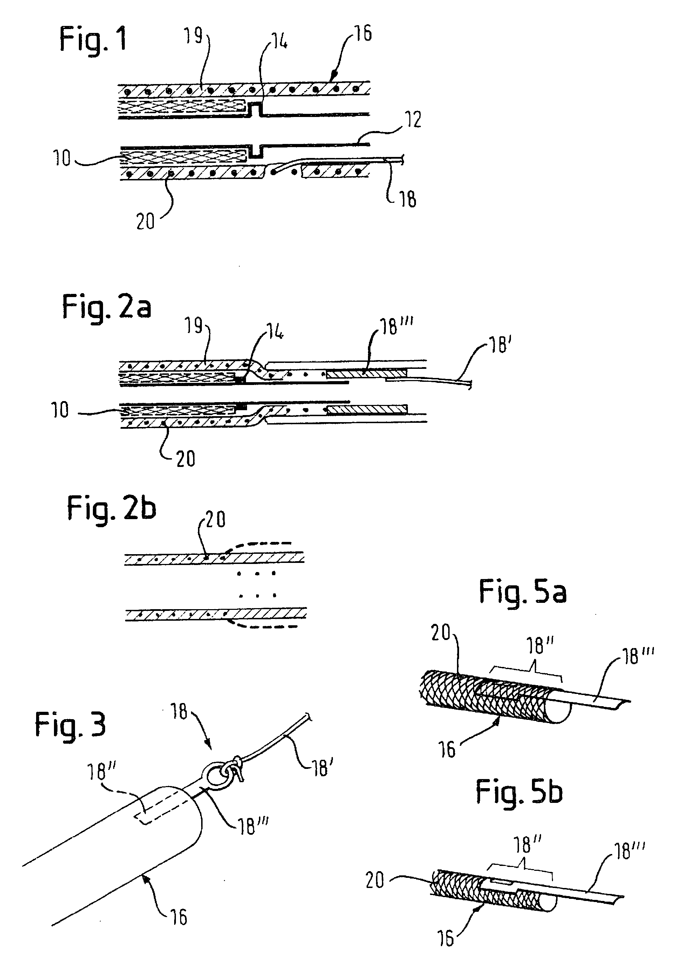

Referring to Fig. 1, a self-expanding implant 10, known as a stent, is placed around a shaft tube 12 and abuts on an abutment member 14 that can be formed integrally with the shaft tube 12 or can be a separate member, as for example a ring clamped or otherwise fixed on the shaft tube. The shaft tube 12 can be strengthened by a supporting member, such as a steel spring wound tightly to resist compression but to allow off-axis flexibility, in particular if the implant is very long.

Around this arrangement is disposed an outer catheter sheath 16 that covers the distal end portion of the shaft tube 12 and the implant 10. The outer catheter sheath 16 extends longitudinally from a distal end in a proximal direction. The outer sheath 16 may extend substantially the whole length of the delivery system, but preferably is not significantly longer than is needed merely to cover the implant 10. A pull element 18, such as a wire, a strand, a tube or another element, capable of transmitting a pull force and flexible enough to follow the catheter path, is connected to the outer sheath 16 at or near its proximal end. The pull element 18 can consist of a single part, or may contain two or more elements, which then are fixed together. The pull element may include a wire, which usually leads to a hand device (not shown) which is responsible for generating the pull force to retract the outer catheter sheath 16 relative to the shaft tube 12.

As shown schematically in Figure 1, pull wire 18 can be connected to a reinforcing structure 20 embedded in the outer catheter sheath, at an exposed portion where the outer catheter sheath bulk material is not formed or is removed to expose the reinforcing structure 20. There are, however, many different possibilities to connect the pull element 18 to the outer sheath 16. Some other exemplary configurations are discussed in the following.

The outer sheath comprises, at least at its distal end, a flexible material 19 such as polyamide, PEBAX and/or PTFE. The flexible material should have characteristics enabling the sheath to be bendable enough for moving through sharp curvatures in a bodily lumen like the vascular system of a patient.

In this flexible material 19 is embedded a reinforcing structure 20 to provide the outer sheath 16 with a better strength. In particular, the reinforcing structure 20 should be strong under tension in the longitudinal direction of the outer sheath 16, and will thus resist necking, whereby its diameter reduces, when pulled longitudinally. The reinforcing structure 20 is made of a relatively inextensible material compared to the flexible material 19, and may be, for example, stainless steel, or carbon fibre or glass fibre compounds. The reinforcing structure 20 is, thus, ideally suited to transmit the load applied by pulling the pull element 18 to the outer sheath 16, along the length thereof. Of course the reinforcing structure 20 has a construction that enables it still to be bent for movement around the above-mentioned sharp curvatures. Preferred reinforcing structures are wound coils, braidings, other net-shaped or cage structures, or just arrangements of connected wires. However, in other examples, the flexible material 19 can be resistant enough lengthwise stretching to work without a reinforcing structure 20 and a reinforcing structure is not necessarily needed.

In embodiments of the first aspect of the invention, the pull element 18 is connected directly to the reinforcing structure 20, which enables the outer sheath 16 to withstand a greater load applied to the sheath 16 through the pull element 18, without risk of breaking the connection between the pull element 18, and the flexible material 19 of the sheath, and facilitating a low-profile interconnection that does not significantly increase the sheath diameter.

A reliable connection method is to connect the pull element onto the braiding thermally. This allows a very well defined connection to be made and reduces the contact area that is needed to create a resilient connection. Therefore, a thermal connection method, like laser, resistance or gap welding, provides a secure connection and can transmit high pull forces. To improve the thermal connection, a support element, such as a weld ring, can be applied to the connection area. For example, a pull element 18 can be welded onto the reinforcing structure 20 by using the weld ring. Using such a supporting element can improve the force distribution around the sheath circumference, since the weld ring can be welded at almost equal distances around its circumference, and can provide force transmission to the reinforcing structure 20 through more than one transmission point. If the spacing of the welding points is generally equal, the force transmission into the reinforcing structure 20 and, thus, in the outer sheath 16 will be generally equal as well, and this can provide a very consistent force transmission and smooth retraction movement of the outer sheath 16.

To connect the pull element 18 to the reinforcing structure 20, with or without thermal connection, the pull element 18 can be at least partially woven into the reinforcing structure 20. For example, a wire pull element can be threaded through a braiding reinforcing structure. Alternatively, instead of or in addition to a thermal or mechanical fixation, the pull element 18 can be adhered directly to the reinforcing structure 20, for example by using adhesive means.

Furthermore, as illustrated in Figure 2a, the pull element 18 may include a connection link 18"' at its distal end, which can be connected to the reinforcing structure 20. A support element can be used in conjunction with the connection link 18''', but the connection link can also provide the function of the support element. Such a connection link could consist of a tube 18''' which is connected at its distal end to the outer sheath 16 and at its proximal end to a wire 18' that leads out of the catheter. Of course, the shape of the connection link 18" is not limited to a tubular shape. Another possible shape of the connection link 18''' is shown in Figure 3, with a connection to pull wire 18' proximal of the flexible material of outer sheath 16.

The pull element 18 is connected to an exposed portion of the reinforcing structure, so that the pull element 18, the connection link 18" and/or the support element (not shown) can be connected directly thereto. Usually, a reinforced outer sheath 16 is formed with the reinforcing structure 20 completely embedded in the flexible material 19 of the outer sheath 16. Therefore, with a preformed reinforced sheath, it is necessary to remove a part of the flexible material 19 to gain access to the reinforcing structure 20 and to make it possible to connect the pull element directly to the reinforcing structure. The removal of the flexible material 19 can be done in different ways, such as by cutting and/or by thermal means, for example welding. A preferred method, however, is to burn a portion of the flexible material 19 off the reinforcing structure 20 using a laser.

Alternatively, an outer sheath 16 might be formed with an exposed part of the reinforcing structure 20 arranged at a proximal end of the outer sheath 16. In one example not forming part of the invention, the reinforcing structure 20 is only partially embedded in the flexible material 19, and has a portion with a smaller or larger diameter than the diameter of the sheath, outside the flexible material of the tubular sheath wall (see Fig. 2b, dotted and dashed lines respectively).

With a portion of the reinforcing structure exposed, it may be desirable to provide an outer layer, to cover the exposed portion, after the pull element 18 has been connected thereto, to render the sheath atraumatic. As further examples, the exposed portion of the reinforcing structure 20 which the pull element is connected to can be covered by applying an extrusion mass onto this portion, by sticking an adhesive tape onto the exposed portion and/or by providing the exposed portion with a cover tube or similar element.

Such shape tape or cover tube could, for example, be made of a shape memory alloy.

The connection link 18'' as well as the support element can be made from different materials known to the skilled person, such as stainless steel, phynox, titanium, shape memory alloy, such as Nitinol, and/or any other material which is biocompatible and has the requisite mechanical properties.

In examples not forming part of the invention, no reinforcing structure 20 is necessarily incorporated in the flexible material 19 of the outer sheath, although by preference such a reinforcing structure may be provided to these examples as well, since the reinforcing structure 20 improves the strength of the outer sheath 16.

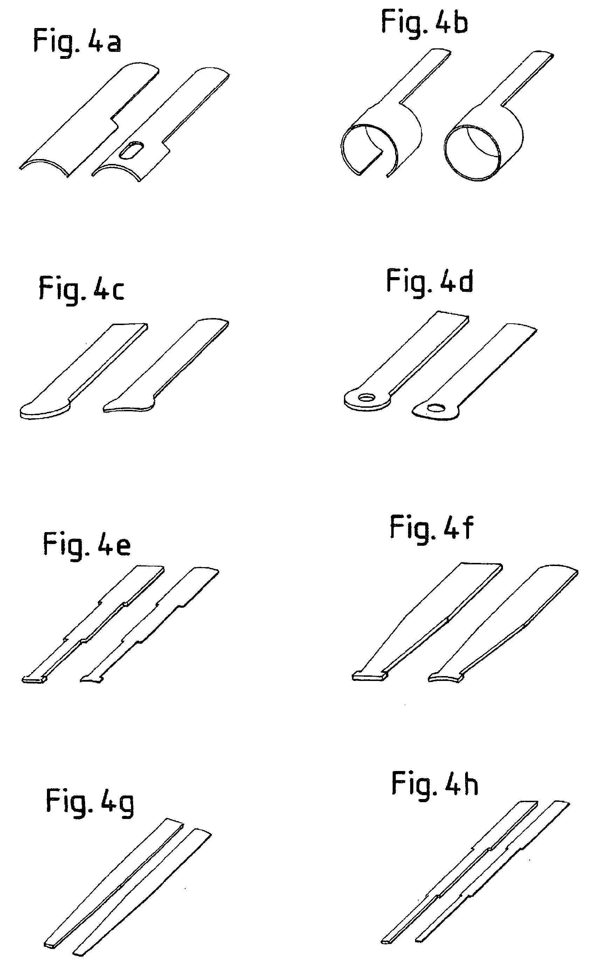

In these examples, the pull element 18 comprises a distal end portion 18'' which has a thickness less than its width and length dimensions and is embedded in the outer catheter sheath 16. The end portion preferably is or includes all or part of a plate member 18", and is disposed in the relatively flexible material 19 of the elongate tubular sheath 16. The end portion may be of many different shapes, as can be seen in the examples of Figures 4a-4h. These shapes can comprise straight, rectangular or stepped sides, as well as tapered sides. A combination of straight and tapered sides is possible as well. Preferably, any end portion can be provided with protruding portions at its distal and/or proximal ends. The protruding portions may protrude left and right (corresponding to the circumferential directions of the tubular sheath wall when embedded therein) as can be seen, for example, in Figures 4e and 4f. The protrusions may also or instead protrude in an upward or downward direction with respect to the lengthwise direction of the end portion 18" (corresponding to a radially outward or inward direction of the tubular sheath wall when embedded therein). Another possible shape is a tubular end portion with a closed circumference or a part tubular end portion with an incomplete circumference. All such plate shapes may exhibit a curvature to match the tubular form of the elongate outer sheath 16.

With specific reference to Figure 4a, there is shown a pair of plate members which can be used to form the end portion of the pull element 18. As seen in Figure 4a, the distal end of the plate member is that at the front-left of the perspective view, whilst the proximal end is shown at the far-right of the perspective view. (The same orientation is shown in Figures 4a to 4d, but is reversed in Figures 4e to 4h, although in fact the plate members may be embedded into the elongate outer sheath 16 either way round, according to the particular application.) The plate member on the left hand side of Figure 4a is formed with a stepped region approximately half way along its length, at which the width of the plate member decreases from being relatively wide at the near, distal end to being relatively thin at the far, proximal end. As can be seen, the plate member is curved about an axis in the longitudinal direction, to match the curvature of the tube wall of the elongate outer sheath 16. The plate member shown on the right hand side of Figure 4a is similarly formed with a stepped width, reducing from a greater width near the distal end to a reduced width in the center and proximal portions. This right hand side plate member is similarly formed with a curvature to match the tubular wall of the elongate outer sheath 16, and is additionally provided with a through-hole in the plate member near to the distal end, through which the relatively flexible material of the sheath outer wall can be bonded between the radially internal and radially external sides of the plate member 18'', when embedded in the outer sheath 16.

Two further plate members are illustrated in Figure 4b, the right hand member having a tubular (complete tubular) distal portion connected to a proximally extending plate having a relatively minor circumferential extent. The plate member on the left hand side of Figure 4b is similarly shaped, except chat the distal tubular portion is only part-tubular, not extending fully around the circumference of the tubular shape corresponding to the wall of the elongate outer sheath 16.

Figures 4c to 4h show pairs of plate members having the same overall shape on their major surfaces, in which the left hand plate member in each illustrated pair is a flat plate member, and in which the right hand plate member in each pair has been formed so as to be curved about its longitudinal axis to match the curvature of the tubular wall of the elongate outer sheath 16. The plate members in Figure 4c are substantially rectangular in shape, having a bulbous rounded distal end attached thereto, projecting partially in the width wise (circumferential) directions. The plate members illustrated in Figure 4d are of substantially the same shape as those in Figure 4c, although with a through-hole formed in the substantially circular distal end portion of the plate member between the upper and lower surfaces corresponding to the radially outer and inner surfaces of the member when it is embedded into the elongate outer sheath 16.

In Figure 4e, the plate members are stepped to have a width dimension that increases between a proximal region and a central region, and again between the central region and a distal region of the plate member. Left and right (circumferentially extending) projections are provided at the proximal end of the plate members to facilitate connection to the proximal portion of the pull element 18, such as a pull wire 18'. The plate members of Figure 4f are similarly provided with projections at the proximal end, and are tapered from a narrow circumferential width at the proximal end to a wider circumferential width near the central portion, which continues to the distal end of the plate member. The plate members shown in Figure 4g have a similar form to those shown in Figure 4f, whilst the plate members shown in Figure 4h have a similar form to the plate members shown in Figure 4e, except that in Figures 4g and 4h the proximal left and right projections are omitted.

On the one hand, the end portions, and in particular those having shapes with a changing width (stepped or tapering shapes), can form part of the unitary pull element 18 that extends to the proximal end of the catheter, in which case no pull wire is required as part of the pull element. In particular, the shapes of Figures 4e - 4h can be adapted to extend to the proximal end of the catheter and to act as the complete pull element 18. On the other hand, the end portions may be connected to a pull wire 18' as an independent member, to form pull element 18. In this case there may be provided an intermediate connecting portion that facilitates the connection of the pull wire 18' to the end portion 18''.

Another possibility for forming the pull element is to provide a wire with a flattened end, and to incorporate the flattened end at least partially in the flexible material of the outer sheath 16 as the end portion 18". The flattened end of the wire, in whole or in part, acts as the end portion, whereas the wire itself is acting as a pull wire 18'.

The end portion 18" may comprise one or more holes or recesses to increase the bond strength between the flexible material 19 and the end portion 18'' embedded therein. This enables the pull element 18 to transmit sufficient force to the sheath 16, even by merely being embedded in the relatively flexible material 19, to enable the sheath 16 to be retracted by pulling on the pull element 18. Another option could be to provide protrusions on the major surfaces (radially inwardly or outwardly facing surfaces) of the end portion, with a similar effect. The holes, the recesses and/or the protrusions can be micro-dimensional. Such micro dimensional holes, recesses and/or protrusions may give the surface a certain surface roughness, or the surface may be otherwise roughened, preferably to have an Ra surface roughness of between 10 µm and 25 µm on the major surfaces of the end portion 18'. By treating the surface structure of the embedded end portion the bond strength between the end portion 18" and the flexible material 19 can be improved, to allow the pull element 18 to impart a higher tensile force to the sheath 16, as described above. For this reason the surfaces of the end portion 18'' can be corrugated, sand plastered and/or edged or otherwise chemically treated or coated, as appropriate.

The shape of the embedded end portion could be of continuous width and continuous thickness, of tapered or stepped width and a continuous thickness, of a continuous width and tapered or stepped thickness, or of tapered and/or stepped width and thickness. The end portion may be so formed by a mechanical press, by rolling, by laser cutting or by grinding, for example.

The material of the pull element and/or of the end portion may comprise stainless steel, plastic, carbon fiber compound and/or glass fiber compound.

If, in the distal end of the catheter sheath 16, there is no reinforcing structure 20 then the end portion 18'' can be simply embedded in the relatively flexible material 19 of the elongate outer catheter sheath 16. If there is provided a reinforcing structure 20 incorporated into the outer catheter sheath 16, the end portion can be placed underneath (radially inside when embedded in the tubular sheath wall) the reinforcing structure 20 or above (radially outside where embedded in the tubular sheath wall) the reinforcing structure 20. These two arrangements are shown in Figures 5a and 5b, respectively. The incorporation of the end portion 18'' into the material 19 of the outer sheath alone, however, is enough to transmit the necessary tensile force to the outer sheath 16 to retract it.

Alternatively, in embodiments of the present invention, the end portion 18" may be connected to the reinforcing structure. For example, the end portions 18'' can be laser, resistance or gap welded to the reinforcing structure 20, and/or the end portion 18" could be woven into the reinforcing structure 20. In one embodiment, the flattened end of a wire can be threaded through the strands of a braided reinforcing structure, as mentioned before.

The surface of the non-embedded parts of the pull element, i.e. not the end portion 18'', is preferably treated to achieve a low friction between those parts of the pull element 18 (particularly any pull wire 18') which lead to the stent deployment actuator (not shown) and the surrounding components of the stent delivery system. Thus, the pull element 18 can be treated with PTFE, such as to form a PTFE coated pull wire.

Preferred methods for manufacturing and assembling the retractable sheaths of the present invention will now be described.

In one preferred method, an outer sheath is provided comprising a reinforcing structure 20 formed in the tubular wall of the outer sheath. A proximal portion of the relatively flexible material forming the wall of the outer sheath 16 is removed to expose a portion of the reinforcing structure 20. The relatively flexible material of the outer sheath 16 is preferably removed by burning it off from the reinforcing structure 20 using a laser. Alternatively, the relatively flexible material may be removed by mechanical or chemical means, or through alternative burning or melting methods. Pull element 18 is then attached to the exposed portion of the reinforcing structure 20, to form a connection directly with the reinforcing structure 20 by which the outer sheath 16 may be retracted by applying a tensile pull force to the pull element 18. In preferred embodiments, where the reinforcing structure 20 and the pull element 18 are both formed from biocompatible metal, the pull element 18 can be laser or spot welded to the reinforcing structure 20.

The pull element 18 may simply be provided in the form of a pull wire 18' to be welded directly to a portion of the reinforcing structure 20. Alternatively, where a connection link 18''' is provided as part of the pull element 18, the pull wire 18' can be connected to the connection link 18''', which is joined to the reinforcing structure 20. Whether a pull wire 18' is connected directly to the reinforcing structure 20 or a connection link 18"' is used, a weld ring may also preferably be used in order to facilitate welding of the pull element 18 to the reinforcing structure 20. This is particularly preferable where the reinforcing structure 20 is metal braiding, as the ends of the different strands of the braiding around the circumference of the reinforcing structure can all be connected to the weld ring so that tensile retraction forces transmitted through the pull element 18 will be distributed and applied relatively evenly around the circumference of the reinforcing structure, and consequently along and through the outer sheath 16.

After the pull element 18 has been connected to the reinforcing structure 20, it may be desirable to cover over the connection region, for example by extruding an outer layer of relatively flexible material to cover the exposed portion of the reinforcing structure 20 and the connection region where the pull element 18 and reinforcing structure 20 are joined. Alternative means may otherwise be provided for rendering the connection atraumatic, for example by providing a supplementary outer sheath layer, such as a shrink tube, to be shrunk down over the outer sheath 16 and pull element 18.

In another preferred method for forming the retractable catheter, a reinforced outer sheath 16 is provided by first forming an inner layer of relatively flexible material about a mandrel, for example by extrusion. Reinforcing structure 20 is then applied onto the inner layer of relatively flexible material along all or a portion of the length of the inner layer. An outer layer of relatively flexible material is then provided to cover the reinforcing structure 20 and the inner layer of relatively flexible material, so as to encapsulate the reinforcing structure 20 between the inner and outer layers which together make up the outer sheath 16. The inner and outer layers of the outer sheath 16 preferably fused together between gaps or interstices in the reinforcing structure 20. The fusion between inner and outer layers may be achieved during the extrusion of the outer layer onto the inner layer and reinforcing structure 20, or maybe achieved through subsequent heat treatment of the laminated structure. Alternatively, the reinforced sheath produced in this manner may be manufactured in a substantially continuous process by co-extruding the inner and outer layers onto the mandrel at the same time as the reinforcing structure 20 is supplied inbetween the two layers. In either case, the mandrel is then removed, for example by allowing it to deflate or contract radially inwardly, to leave the enforced sheath structure. This sheath structure is treated according to the method above, by removing part of the inner or outer layers to expose a portion of the reinforcing structure 20 to connect it to a pull element 18.

In general, the same relatively flexible material may be used for the inner and outer layers of the outer sheath 16, or two different relatively flexible materials may be used for the respective layers.

## Claims

1. A retractable catheter preferably for use in an implant delivery catheter system, the catheter comprising:
an elongate tubular sheath (16) which is at least in a distal portion made of a relatively flexible material (19);
a reinforcing structure (20) which is made of a relatively inextensible material and which is incorporated, along at least a portion of the length of the sheath, in the relatively flexible material of the sheath; and
a pull element (18) for transmitting a pull force longitudinally to the sheath and the reinforcing structure,
**characterized in that**:
the pull element (18) is connected directly to a portion of the reinforcing structure (20) exposed from the relatively flexible material (19), wherein the exposed portion of the reinforcing structure is exposed in an opening in the flexible material, to transmit the pull force directly to the reinforcing structure (20).

2. A retractable catheter according to claim 1, wherein the reinforcing structure (20) comprises a braiding.

3. A retractable catheter according to claim 1 or 2, wherein the pull element (18) is thermally connected onto the reinforcing structure (20), the thermal connection method preferably including laser welding, resistance welding, gap welding, and/or point welding.

4. A retractable catheter according to any preceding claim, wherein the pull element (18) is provided with at least one connection link (18'', 18''') by which it is connected to the reinforcing structure (20).

5. A retractable catheter according to claim 4, wherein the connection link (18'', 18''') has an at least partially tubular element to support the connection to the reinforcing structure (20).

6. A retractable catheter according to any preceding claim, wherein at least a portion of the pull element (18) is partially tubular, preferably at its distal end.

7. A retractable catheter according to any one of claims 1 to 5, wherein the pull element (18) is, at least at its distal end, tube shaped.

8. A retractable catheter according to any preceding claim, wherein the reinforcing structure (20) and/or the pull element (18) and/or the connection link (18'', 18''') comprise steel, stainless steel, plastic, a carbon fibre compound and/or a glass fibre compound.

9. An implant delivery system comprising a retractable catheter (16) according to any preceding claim, the delivery system further comprising an inner catheter shaft (12) and a implant (10).

10. A method for assembling a retractable catheter for use in an implant delivery system, comprising the step of:
- providing a catheter sheath (16) made of relatively flexible material (19) in which a relatively inextensible reinforcing structure (20) is incorporated, and
**characterized by** the steps of:
- partially removing the relatively flexible material (19) of the catheter sheath to expose a portion of the reinforcing structure (20) to which a pull element (18) is to be directly connected; and
- connecting the pull element (18) directly to the exposed portion of the reinforcing structure (20).

11. A method according to claim 10, wherein the removal of the relatively flexible material (19) is by thermal means, preferably using a laser.

12. A method according to claim 10 or 11, wherein the method further comprises the steps of:
- providing the pull element (18) with a connection link (18'', 18'''); and
- connecting the connection link of the pull element directly to the reinforcing structure (20).

13. A method according to any one of claims 10 to 12, wherein the connection of the pull element (18) to the reinforcing structure (20) is made by laser welding and/or point welding.

14. A method according to any one of claims 10 to 13, further comprising the step of:
- covering the exposed portion of the reinforcing structure (20) after the connection of the pull element (18) thereto.

15. A method according to any one of claims 10 to 14, to assemble a retractable catheter according to any one of claims 1 to 8.

## Patentansprüche

1. Zurückziehbarer Katheter, bevorzugt zur Verwendung in einem Implantatzuführkathetersystem, der Katheter mit:
einer länglichen röhrenförmigen Hülse (16), die zumindest in einem distalen Abschnitt aus einem relativ flexiblen Material (19) hergestellt ist;
einer Verstärkungsstruktur (20), die aus einem relativ undehnbaren Material hergestellt ist und die entlang zumindest eines Abschnitts der Länge des Hülse in dem relativ flexiblen Material der Hülse eingearbeitet ist; und
einem Zugelement (18) zum Übertragen einer Zugkraft in Längsrichtung zu der Hülse und der Verstärkungsstruktur,
**dadurch gekennzeichnet, dass**:
das Zugelement (18) direkt mit einem Abschnitt der Verstärkungsstruktur (20) verbunden ist, der freistehend von dem relativ flexiblen Material (19) ist, wobei der freistehende Abschnitt der Verstärkungsstruktur in einer Öffnung in dem flexiblen Material freistehend ist, um die Zugkraft direkt zu der Verstärkungsstruktur (20) zu übertragen.

2. Rückziehbarer Katheter nach Anspruch 1, bei dem die Verstärkungsstruktur (20) ein Geflecht aufweist.

3. Rückziehbarer Katheter nach Anspruch 1 oder 2, bei dem das Zugelement (18) auf der Verstärkungsstruktur (20) thermisch verbunden ist, wobei das thermische Verbindungsverfahren bevorzugt Laserschweißen, Widerstandsschweißen, Spaltschweißen und/oder Punktschweißen einschließt.

4. Rückziehbarer Katheter nach einem der vorstehenden Ansprüche, bei dem das Zugelement (18) mit mindestens einem Verbindungsglied (18'', 18''') bereitgestellt ist, durch das es mit der Verstärkungsstruktur (20) verbunden ist.

5. Rückziehbarer Katheter nach Anspruch 4, bei dem das Verbindungsglied (18'', 18''') ein zumindest teilweise röhrenförmiges Element aufweist, um die Verbindung mit der Verstärkungsstruktur (20) zu unterstützen.

6. Rückziehbarer Katheter nach einem der vorstehenden Ansprüche, bei dem mindestens ein Abschnitt des Zugelements (18) abschnittsweise röhrenförmig ist, und zwar bevorzugt an seinem distalen Ende.

7. Rückziehbarer Katheter nach einem der Ansprüche 1 bis 5, bei dem das Zugelement (18) zumindest an seinem distalen Ende eine Röhrenform aufweist.

8. Rückziehbarer Katheter nach einem der vorstehenden Ansprüche, bei dem die Verstärkungsstruktur (20) und/oder das Zugelement (18) und/oder das Verbindungsglied (18'', 18''') Stahl, rostfreien Stahl, Kunststoff, einen Kohlefaserverbund und/oder einen Glasfaserverbund aufweist.

9. Implantatzuführsystem mit einem rückziehbaren Katheter (16) nach einem der vorstehenden Ansprüche, das Zuführsystem des Weiteren mit einem inneren Katheterschaft (12) und einem Implantat (10).

10. Verfahren zum Zusammenbau eines rückziehbaren Katheters zur Verwendung in einem Implantatzuführsystem, mit den Schritten:
- Bereitstellen einer Katheterhülse (16), die aus einem relativ flexiblen Material (19) hergestellt ist, in dem eine relativ undehnbare Verstärkungsstruktur (20) eingearbeitet ist, und
**gekennzeichnet durch** die Schritte:
- teilweises Entfernen des relativ flexiblen Materials (19) von der Katheterhülse, um einen Abschnitt der Verstärkungsstruktur (20), mit dem ein Zugelement (18) direkt zu verbinden ist, freizulegen; und
- direktes Verbinden des Zugelements (18) mit dem freigelegten Abschnitt der Verbindungsstruktur (20).

11. Verfahren nach Anspruch 10, bei dem das Entfernen des relativ flexiblen Materials (19) durch ein thermisches Mittel erfolgt, vorzugsweise unter Verwendung eines Lasers.

12. Verfahren nach Anspruch 10 oder 11, bei dem das Verfahren des Weiteren die Schritte umfasst:
- Bereitstellen des Zugelements (18) mit einem Verbindungsglied (18'', 18'''); und
- direktes Verbinden des Verbindungsglieds von dem Zugelement mit der Verstärkungsstruktur (20).

13. Verfahren nach einem der Ansprüche 10 bis 12, bei dem die Verbindung des Zugelements (18) mit der Verstärkungsstruktur (20) durch Laserschweißen und/oder Punktschweißen erzeugt wird.

14. Verfahren nach einem der Ansprüche 10 bis 13, des Weiteren mit dem Schritt:
- Abdecken des freigelegten Abschnitts der Verstärkungsstruktur (20) nach der Verbindung des Zugelements (18) damit.

15. Verfahren nach einem der Ansprüche 10 bis 14, um einen rückziehbaren Katheter nach einem der Ansprüche 1 bis 8 zu montieren.

## Revendications

1. Cathéter rétractable à utiliser de préférence dans un système de cathéter pour pose d'implant, le cathéter comprenant :
une gaine tubulaire allongée (16) qui est au moins dans une partie distale réalisée en un matériau relativement flexible (19) ;
une structure de renfort (20) qui est réalisée en un matériau relativement non extensible et qui est incorporée, le long d'au moins une partie de la longueur de la gaine, dans le matériau relativement flexible de la gaine ; et
un élément de traction (18) pour transmettre une force de traction longitudinalement à la gaine et à la structure de renfort, **caractérisé en ce que** :
l'élément de traction (18) est directement raccordé à une partie de la structure de renfort (20) exposée depuis le matériau relativement flexible (19), où la partie exposée de la structure de renfort est exposée dans une ouverture dans le matériau flexible, pour transmettre directement la force de traction à la structure de renfort (20).

2. Cathéter rétractable selon la revendication 1, dans lequel la structure de renfort (20) comprend une tresse.

3. Cathéter rétractable selon la revendication 1 ou 2, dans lequel l'élément de traction (18) est thermiquement raccordé à la structure de renfort (20), le procédé de raccord thermique comprenant de préférence la soudure par laser, la soudure par résistance, la soudure à écartement, et/ou la soudure par point.

4. Cathéter rétractable selon l'une des revendications précédentes, dans lequel l'élément de traction (18) est doté d'au moins un lien de raccord (18'', 18''') par lequel il est raccordé à la structure de renfort (20).

5. Cathéter rétractable selon la revendication 4, dans lequel le lien de raccord (18", 18''') présente un élément au moins partiellement tubulaire pour supporter le raccord à la structure de renfort (20).

6. Cathéter rétractable selon l'une des revendications précédentes, dans lequel au moins une partie de l'élément de traction (18) est partiellement tubulaire, de préférence à son extrémité distale.

7. Cathéter rétractable selon l'une quelconque des revendications 1 à 5, dans lequel l'élément de traction (18) est, au moins à son extrémité distale, en forme de tube.

8. Cathéter rétractable selon l'une des revendications précédentes, dans lequel la structure de renfort (20) et/ou l'élément de traction (18) et/ou le lien de raccord (18'', 18''') sont en acier, en acier inoxydable, en plastique, d'un composé de fibre de carbone et/ou d'un composé de fibre de verre.

9. Système de pose d'implant comprenant un cathéter rétractable (16) selon l'une des revendications précédentes, le système de pose comprenant en outre une tige de cathéter interne (12) et un implant (10).

10. Procédé d'assemblage d'un cathéter rétractable à utiliser dans un système de pose d'implant, comprenant l'étape consistant :
- à fournir une gaine de cathéter (16) réalisée en un matériau relativement flexible (19) dans lequel une structure de renfort relativement inextensible (20) est incorporée, et
**caractérisé par** les étapes consistant :
- à éliminer partiellement le matériau relativement flexible (19) de la gaine de cathéter pour exposer une partie de la structure de renfort (20) à laquelle un élément de traction (18) doit être directement raccordé ; et
- à raccorder directement l'élément de traction (18) à la partie exposée de la structure de renfort (20).

11. Procédé selon la revendication 10, dans lequel l'enlèvement du matériau relativement flexible(19) est réalisé par un moyen thermique, de préférence en utilisant le laser.

12. Procédé selon la revendication 10 ou 11, dans lequel le procédé comprend en outre les étapes consistent :
- à doter l'élément de traction (18) d'un lien de raccord (18', 18''') ; et
- à raccorder directement le lien de raccord de l'élément de traction à la structure de renfort (20).

13. Procédé selon l'une quelconque des revendications 10 à 12, dans lequel le raccord de l'élément de traction (18) à la structure de renfort (20) est réalisé par soudure au laser et/ou soudure par point.

14. Procédé selon l'une quelconque des revendications 10 à 13, comprenant en outre l'étape consistent :
- à recouvrir la partie exposée de la structure de renfort (20) après le raccord de l'élément de traction (18) à celle-ci.

15. Procédé selon l'une quelconque des revendications 10 à 14, pour assembler un cathéter rétractable selon l'une quelconque des revendications 1 à 8.
